Europäisches Patentamt

(19) European Patent Office

Office européen des brevets

(11) Publication number: **0 259 246**
A2

(12) **EUROPEAN PATENT APPLICATION**

(21) Application number: 87630173.0

(22) Date of filing: 01.09.87

(51) Int. Cl.⁴: **C 08 J 5/18**
C 08 L 23/08, A 61 L 15/00,
A 41 B 13/02

(30) Priority: 05.09.86 US 903933

(43) Date of publication of application:
09.03.88 Bulletin 88/10

(84) Designated Contracting States:
AT BE CH DE ES FR GB GR IT LI LU NL SE

(71) Applicant: JAMES RIVER CORPORATION
One Bush Street
San Francisco California 94104 (US)

(72) Inventor: Zuscik, Edward J.
37 Bay Forest Court
Oakland California 94611 (US)

(74) Representative: Schmitz, Jean-Marie et al
OFFICE DENNEMEYER S.à.r.l. P.O. Box 1502
L-1015 Luxembourg (LU)

(54) Film for forming shirred articles.

㊵ Elastic waist bands for disposable diapers can be made from a blown heat-shrinkable film comprising a blend of from about 70 to 90 percent of an ethylene-vinyl acetate elastomer and 10 to 30 percent of a nonelastomeric polyolefin, such as linear low density polyethylene. The film, which has a relatively high degree of orientation has good shrinkage and good elastic force.

EP 0 259 246 A2

## Description

This invention relates to a blown film comprising a blend of an ethylene-vinyl acetate copolymer and a polyolefin. The film is especially suitable for forming shirred articles.

US patent 3 175 027, 3 245 407, 3 639 917 and 3 819 401 describe a method of preparing shirred articles, such a disposable garments, by adhering a band of a heat shrinkable elastomer to a web of flexible material and heating the band to cause the article to shirr or gather at the location of the band. The band, being elastic, causes the article to fit snugly on the wearer, such as at the waist. A particular application of this method is shown in US patent 4 563 185, which describes the manufacture of disposable diapers wherein segments of a heat-shrinkable film are adhered to the backsheet of the diapers and heated to form elastic waist bands. The film and the backsheet are supplied continuously in the machine direction (MD), so the desired direction of shrinkage of the segments is in the cross direction (CD). The film was produced by extruding a film having a thickness of 6 mils and then orienting it to a thickness of 1.5 mils on a tenter frame orienter. The orientation was needed to impart sufficient shrink force to the film so that, when heat shrunk, it would gather the diaper at the waist.

US patent 4 476 180 discloses disposal diapers having an elastic waist band made from a film comprising a blend of an ethylene-vinyl acetate copolymer and another elastomer, but the film is a cast film and therefore it would not have sufficient CD shrinkage to be utilized in the method described above. US patent 4 504 434 discloses brown films comprising a blend of an ethylene-vinyl acetate copolymer and a polyolefin, but the films were made at a low blow-up ratio and therefore would also have insufficient CD heat shrinkage.

This invention is an improved heat-shrinkable film having both good elasticity and strong CD shrink force. The film is made in a one step process. A secondary orientation process is not required. The film is particu larly suitable for forming shirred articles, particularly disposable diapers made in accordance with the method described in US patent 4 563 185. The film comprises from about (a) 60 to 95 percent ethylene-vinyl acetate copolymer wherein the vinyl acetate content is about 25 to 29 percent and (b) about 5 to 40 percent of a nonelastomeric polyolefin containing up to ten percent of an ethylenically-unsaturated comonomer randomly polymerized therewith. The film is oriented by being blown at a relatively high blow-up ratio and at an extrusion temperature that is preferably between about 140 and 190°C. The blown film has, after heat shrinkage, sufficient shrinkage in the cross direction and sufficient elastic force to be suitable for forming shirred articles, especially disposable diapers.

### Free Shrinkage -
A 4.5″ diameter sample is submerged in an oil bath at 180°F (82°C) for 30 seconds and then quenched in tap water. The diameter of the shrunken sample is measured in the machine direction and cross machine direction.

### Shrinkage under Tension -
A one-inch wide sample is held horizontally in the cross direction between two clips set six inches apart. One clip is stationary and the other is attached to a 30 gram weight that rests on a metal plate. The weight is 1-9/16″ long by 1/2″ wide × 1/2″ high. The long dimension of the weight corresponds to the long dimension of the sample. The weight and the metal plate are covered with Teflon tape so the weight is free to move. The apparatus is placed in a circulating air oven at 200°F (93°C) for one minute and the amount of shrinkage measured. The test simulates the shrinkage obtained with heat-shrinkable waist bands in disposable diapers of the type described in US patent 4 563 185.

### Elastic Force -
A film sample that has been heat shrunk by either of the above methods is extended from 1.25″ to 3.75″ at ambient temperature and held at that extension for one minute. The forces required to hold the sample at that extension initially and after one minute are determined. The forces are the initial Elastic Force and residual Elastic force, respectively.

### Shrink Force -
A one inch wide sample is placed between jaws in an Instron tensile tester that is in a temperature control cabinet. The jaws are two inches apart. The cabinet temperature is raised from ambient to 200°F at about 25°F per minute. The jaws are kept stationary and the force on the jaws is monitored. The force at various temperatures is reported.

### Force at 100 % Elongation -
The force required to extend a film sample to 100 % elongation is determined.

### Other Properties -
Tensile strength, yield stress, ultimate elongation and secant modulus are tested under ASTM D882. Density is measured in a density gradient column. Basis weight is measured on an analytical balance using a 4″ × 4″

sample and converted to pounds per 3000 sq. ft. ream. Thickness in mils is calculated from the density and basis weight by using the formula thickness = 0.064 × basis weight/density. Melt index is measured under ASTM D1238-82.

The film of this invention preferably comprises from about 70 to 90 percent of the ethylene-vinyl acetate copolymer and correspondingly from about 10 to 30 percent of the nonelastomeric polyolefin. This proportion of the polyolefin is preferred because at lower levels the Shrinkage Under Tension is low and at higher levels the film is not sufficiently elastic.

As used herein, the term nonelastomeric polyolefin refers to a polymer having a secant modulus greater than about 15,000 psi when made into blown film. Suitable nonelastomeric polyolefins include low density ethylene homopolymer, linear low density polyethylene, ionomers, and ethylene-vinyl acetate copolymers wherein the vinyl acetate content is less than about 8 percent. The non-elastomeric polyolefin is preferably linear low density polyethylene (LLDPE). LLDPE has a molecular structure which is characterized by the substantial absence of long chain branching. In contrast, conventional low density polyethylene has substantial long chain branching. LLDPE also has a significantly higher melting point (typically 120-135°C) than conventional low density polyethylene (typically 105-115°). Conventional low density polyethylene is sometimes referred to as high pressure polyethylene because it is produced at high pressures. LLDPE, on the other hand, is produced commercially at low pressures in a gas phase process. However, LLDPE may also be produced in a liquid phase solution process. Various alpha-olefins are typically copolymerized with ethylene in producing LLDPE. The alpha-olefins, which preferably have 4 to 8 carbon atoms, are present in the polymer in an amount of up to about ten percent by weight. The most typical comonomers are butene, hexene, and octene. The comonomer influences the density of the polymer, which is preferably less than about 0.955 grams per cubic centimeter.

The melt index of the nonelastomeric polyolefin is preferably between about 0.25 and 6, more preferably between about 0.5 and 1.0. The melt index of the ethylene-vinyl acetate coplymer is preferably between about 0.5 and 6, more preferably between about 1 and 2.

The Shrinkage Under Tension of the films of this invention is preferably at least about twenty percent.

The initial Elastic Force is preferably at least 480 grams/inch/mil based on the initial width and thickness. The residual elastic force is at least sixty percent of the initial Elastic Force.

The Force at 100% Elongation is preferably between about 300 and 550 grams/inch.

The stiffness (secant modulus) of the films is preferably between about 3,000 and 10,000 psi, more preferably between about 3,000 and 8,000 psi.

The degree of orientation imparted to the film as it is blown is important in attaining the desired properties of the film. When blowing is initiated below the frost line of the film, which is the typical method in the art, the blow up ratio is preferably at least 3.2, which is substantially greater than the typical blow up ratio. However, as appreciated by those skilled in the art, the same or greater degree of orientation may be obtained at a lower blow up ratio by utilizing special means to lower the temperature of the film before blowing it.

The film of this invention may be adhered to a web of flexible material and heated to form a shirred article in accordance with methods known in the art. The film is especially suitable for forming an elastic waist band of a disposable diaper, such as in accordance with US patent 4 563 185 which is incorporated herein by reference. The preferred thickness of the film for this purpose is between about 0.8 and three mils. The film is preferably heated to a temperature between about 60 and 95°C to cause shirring of the article. Higher temperatures are undesirable as they tend to cause deterioration of other properties of the diaper.

All percentages referred to herein are by weight.

Example 1

A resin blend was extruded into a blown film using a 3.5-inch extruder (24:1 L/D ratio) and ten-inch diameter blown film die. The bubble diameter was 35 inches so the blow up ratio was 3.5. The temperature of the melt from the extruder was between 180 and 210°C and the temperature of the die was between 160 and 180°C. The blend consisted of 69 percent of an ethylene-vinyl acetate copolymer having a vinyl acetate content of 28 percent and a melt index of 3 (Norchem 3305), 16 percent of polyethylene having a density of 0.924 and a melt index of 0.8 (Chevron 5036), 10 percent of an ionomer having a melt index of 5.5 (Dupont 1705 Surlyn), 3 percent of Dupont 9619 (ethylene-vinyl acetate copolymer having a vinyl acetate content of 18 percent and containing 8 percent of a slip agent and 20 percent of an antiblock agent), and 2 percent of Color Chip 88170 (low density polyethylene containing a blue pigment). The properties of the film are reported in Table 1.

Example 2

Example 1 was repeated except the melt temperature was 205°C, the die temperature was 140 to 150°C, and the blend consisted of 76 percent of an ethylene-vinyl acetate copolymer having a vinyl acetate content of 28 percent and a melt index of 1.8 (Norchem 3301), 20 percent of linear low density polyethylene having a density of 0.920 and a melt index of 0.8 (Dow 61508.13), 2 percent of DuPont 9619 and 2 percent of Color Chip 88170. The properties of the film are reported in Table 1.

**0 259 246**

TABLE 1

|  | | Example 1 | Example 2 |
|---|---|---|---|
| Thickness, mils | | 1.15 | 1.25 |
| Density, g/cc | | 0.950 | 0.945 |
| Tensile Yield, psi : | MD | 600 | 430 |
| | CD | 400 | 430 |
| Tensile Break, psi : | MD | 2800 | 4480 |
| | CD | 3000 | 4950 |
| Ultimate Elongation, % : | MD | 400 | 570 |
| | CD | 550 | 570 |
| Force at 100 % Elongation, grams | | 410 | NM |
| Shrink Force at $140^{o}$C | | 7.6 | NM |
| Secant Modulus, psi : | MD | 8000 | 5600 |
| | CD | 7000 | 6500 |
| Free Shrinkage, % : | MD | 8 | 3.5 |
| | CD | 22 | 20 |
| Elastic Force, initial, grams | | 600 | 680 |
| Elastic Force, residual, grams | | 370 | 460 |
| Shrinkage Under Tension | | NM | 23.5 |

NM = not measured

As indicated by the results reported in the Table, the films had both good shrinkage and good elasticity, which makes them especially suitable for forming the waist band of disposable diapers. Good shrinkage is required in order to form a good gathered waist at the high production speeds employed in the manufacture of the diapers. Good elasticity is required so the waist band can be expanded without much force. These requirements naturally compete with each other, but the films of this invention satisfy both requirements with excellent results and they are made in a one step process that does not require a secondary (e.g. tenter frame) orientation process.

**Claims**

1. A heat-shrinkable blown film suitable for forming a shirred article, said film having a Shrinkage Under Tension of more than twenty percent, a stiffness between 3000 and 10000 psi, and after heat shrinkage, an initial Elastic Force of at least 480 grams/inch/mil and a residual Elastic Force equal to at least sixty percent of the initial Elastic Force, said film comprising (a) from about 60 to 95 percent of an ethylene-vinyl acetate copolymer wherein the vinyl acetate content is about 25 to 29 percent and (b) from about 5 to 40 percent of a nonelastomeric polyolefin containing up to ten percent of an ethylenically-unsaturated comonomer randomly polymerized therewith.

2. The film of claim 1 wherein the nonelastomeric polyolefin is linear low density polyethylene.

3. The film of claim 1 wherein the film comprises from about 70 to 90 percent of the ethylene-vinyl acetate copolymer and from about 10 to 30 percent of the nonelastomeric polyolefin.

4. The film of claim 3 wherein the melt index of the ethylene-vinyl acetate copolymer is between about 0.5 and 6 and the melt index of the nonelastomeric polyolefin is between about 0.25 and 6.

5. The film of claim 4 having a thickness between about 0.8 and three mils.

6. A shirred article comprising the film of claim 1 adhered to a web of flexible material, said article having been heated to a temperature sufficient to cause the film to shrink and thereby shirr the article.

7. The article of claim 6 wherein the article is a disposable diaper.

8. The article of claim 7 wherein the film forms an elastic waist band.

4